(19) Europäisches Patentamt
European Patent Office
Office européen des brevets

(11) **EP 3 821 799 B1**

(12) **EUROPEAN PATENT SPECIFICATION**

(45) Date of publication and mention
of the grant of the patent:
**20.11.2024   Bulletin 2024/47**

(21) Application number: **18925916.1**

(22) Date of filing: **12.07.2018**

(51) International Patent Classification (IPC):
**A61B 5/276** (2021.01)     **A61B 5/28** (2021.01)
**A61B 5/318** (2021.01)     **A61B 5/305** (2021.01)
**A61B 5/00** (2006.01)

(52) Cooperative Patent Classification (CPC):
**A61B 5/276; A61B 5/28; A61B 5/305; A61B 5/318;**
A61B 5/7203; A61B 5/7225

(86) International application number:
**PCT/CN2018/095458**

(87) International publication number:
**WO 2020/010580 (16.01.2020 Gazette 2020/03)**

(54) **THREE-LEAD ELECTROCARDIOGRAPHY MONITORING METHOD AND DEVICE**

VERFAHREN UND VORRICHTUNG ZUR ÜBERWACHUNG EINES
3-KANAL-ELEKTROKARDIOGRAMMS

PROCÉDÉ ET DISPOSITIF DE SURVEILLANCE D'ÉLECTROCARDIOGRAPHIE À TROIS
DÉRIVATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**19.05.2021   Bulletin 2021/20**

(73) Proprietors:
• **Shenzhen Mindray Bio-Medical Electronics Co.,
Ltd.**
**Shenzhen, Guangdong 518057 (CN)**
• **Shenzhen Mindray Scientific Co., Ltd.**
**Shenzhen, Guangdong 518000 (CN)**

(72) Inventors:
• **LIU, Qiling**
**Shenzhen, Guangdong 518057 (CN)**
• **REN, Jian**
**Shenzhen, Guangdong 518057 (CN)**
• **XUE, Bingbing**
**Shenzhen, Guangdong 518057 (CN)**
• **JIAO, Kun**
**Shenzhen, Guangdong 518057 (CN)**
• **CEN, Jian**
**Shenzhen, Guangdong 518057 (CN)**
• **ZHANG, Bo**
**Shenzhen, Guangdong 518057 (CN)**

(74) Representative: **KIPA AB**
**Drottninggatan 11**
**252 21 Helsingborg (SE)**

(56) References cited:
EP-A1- 0 835 635     CN-A- 103 027 675
CN-A- 103 860 163    CN-A- 105 342 601
CN-A- 107 456 230    CN-U- 205 251 532
US-A1- 2007 038 257  US-A1- 2010 152 596
US-A1- 2010 152 596  US-A1- 2015 327 815

## Description

## TECHNICAL FIELD

The disclosure relates to the field of medical monitoring, and in particular to a three-lead electrocardiography monitoring method and device.

## BACKGROUND

A monitoring device with an electrocardiogram (ECG) monitoring function generally needs to use a right leg drive circuit because: (1) an ECG signal is very weak (usually with only a few microvolts); and (2) the body of a monitored person (for example, a patient) may also be interfered as an antenna, especially interfered by a power frequency of 50 Hz or 60 Hz, which may cover an ECG signal of the patient. Therefore, the two factors (1) and (2) may cause measurement of the ECG signal of the patient being difficult. However, the use of the right leg drive circuit may improve a common-mode rejection ratio of a collection system, thereby reducing an amplitude of a common-mode interference signal in a collected ECG signal, and greatly reducing the above power frequency interference.

A typical three-lead electrocardiography collection circuit with a right leg drive circuit is used as an example. Three leads are three limb leads (lead labels are respectively RA, LA, and LL), comprising two measurement-leads and one driving-lead. When the driving-lead is any one (for example, LL) of RA, LA, or LL, the remaining two leads (for example, RA and LA) are measurement-leads. Voltages of two measurement-leads connected to a human body are processed by the right leg drive circuit, and then connected to the human body as a common-mode driving signal of the human body by means of the driving-lead, and then a differential voltage of the above two measurement-leads is used as an EGC signal of one channel.

Since the above three-lead electrocardiography monitoring device can support only ECG waveform collection and analysis on one channel, there is a relatively high probability of errors in the final electrocardiography parameter and a further electrocardiography analysis result (for example, arrhythmia).

EP0835635 discloses an automatic lead switching for ECG monitor. The monitor can determine whether the reference electrode has been disconnected and if so, switch to another reference electrode, which is selected in accordance with a predetermined priority scheme, to continue ECG monitoring.

US20100152596 discloses an apparatus for detecting the position of a catheter apparatus for detecting the position of a catheter. The controller of the apparatus has at least one output connectable to an ECG patient connector, where the controller includes at least one input and the at least one input includes an electrical connection from the controller to the output of the connector.

US20070038257 discloses an apparatus for accessing the electrical properties of patient electrode interfaces, and the electrode interface quality can be displayed in a useful manner and used to carry out electrode lead switching.

## SUMMARY

In view of the above situation, the disclosure provides a three-lead electrocardiography monitoring method and device.

According to a first aspect, an embodiment provides a three-lead electrocardiography monitoring method, where the three leads may include two measurement-lead electrodes and one driving-lead electrode that are attached to a measured object, and the method may include: acquiring an electrocardiography signal of a first channel of the measured object by means of the two measurement-lead electrodes; acquiring an electrocardiography signal of a second channel of the measured object by means of either of the measurement-lead electrodes and the driving-lead electrode. The method may further include acquiring a common-mode interference amplitude in an electrocardiography signal of the measured object by means of one or both of the two channels, and choosing one or both of the acquired electrocardiography signal of the first channel and the electrocardiography signal of the second channel of the measured object for processing, to obtain an electrocardiography parameter of the measured object.

In an embodiment, said choosing to acquire one or both of the electrocardiography signal of the first channel and the electrocardiography signal of the second channel of the measured object for processing comprises:

choosing to acquire the electrocardiography signal of the first channel of the measured object for processing; or
choosing to acquire the electrocardiography signal of the first channel and the electrocardiography signal of the second channel of the measured object for processing.

In an embodiment, said choosing to acquire one or both of the electrocardiography signal of the first channel and the electrocardiography signal of the second channel of the measured object for processing comprises:

acquiring a common-mode interference amplitude in an electrocardiography signal of the measured object by means of one or both of the two channels; and
choosing, according to the common-mode interference amplitude, to acquire one or both of the electrocardiography signal of the first channel and the electrocardiography signal of the second channel of the measured object for processing.

According to a second aspect, an embodiment provides a three-lead electrocardiography monitoring device, comprising:

two measurement-lead electrodes and one driving-lead electrode that are attached to a measured object;
a first channel circuit connected to the two measurement-lead electrodes and configured to acquire an electrocardiography signal of a first channel of the measured object by means of the two measurement-lead electrodes;
a second channel circuit connected to either of the measurement-lead electrodes and the driving-lead electrode, and configured to acquire an electrocardiography signal of a second channel of the measured object by means of the measurement-lead electrode and the driving-lead electrode that are connected to the second channel circuit; and
a processor configured to acquire a common-mode interference amplitude in an electrocardiography signal of the measured object by means of one or both of the two channels, and choose, according to the common-mode interference amplitude, one or both of the acquired electrocardiography signal of the first channel and the electrocardiography signal of the second channel of the measured object for processing, to obtain an electrocardiography parameter of the measured object.

In an embodiment, the processor chooses to acquire the electrocardiography signal of the first channel of the measured object for processing, or chooses to acquire the electrocardiography signal of the first channel and the electrocardiography signal of the second channel of the measured object for processing.

In an embodiment, the processor acquires a common-mode interference amplitude in an electrocardiography signal of the measured object by means of one or both of the two channels, and chooses, according to the common-mode interference amplitude, to acquire one or both of the electrocardiography signal of the first channel and the electrocardiography signal of the second channel of the measured object for processing.

According to a third aspect, an embodiment provides a computer-readable storage medium, which comprises a program that can be executed by a processor to implement the method in any one of the above embodiments.

According to the three-lead electrocardiography monitoring method and device, and the computer-readable storage medium in the above embodiments, the electrocardiography signal of the first channel of the measured object is acquired by means of the two measurement-lead electrodes, and the electrocardiography signal of the second channel of the measured object is acquired by means of either of the measurement-lead electrodes and the driving-lead electrode, thereby implementing electrocardiography signal collection and analysis on two channels under three-lead electrocardiography monitoring, and improving accuracy of the final electrocardiography parameter and a further electrocardiography analysis result.

## BRIEF DESCRIPTION OF THE DRAWINGS

FIG. 1 is a schematic structural diagram of a multi-parameter monitoring device according to an embodiment;
FIG. 2 is a schematic structural diagram of a single-parameter monitoring device according to an embodiment;
FIG. 3 is a schematic structural diagram of a monitoring device networking system in a hospital according to an embodiment;
FIG. 4 is a schematic structural diagram of a three-lead electrocardiography monitoring device according to an embodiment;
FIG. 5 is a schematic structural diagram of a three-lead electrocardiography monitoring device according to another embodiment;
FIG. 6 (a), FIG. 6 (b), and FIG. 6 (c) are respectively schematic structural diagrams of three three-lead electrocardiography monitoring devices comprising right leg drive circuits;
FIG. 7 is an equivalent circuit diagram of a three-lead electrocardiography monitoring device according to an embodiment;
FIG. 8 is a flowchart of a three-lead electrocardiography monitoring method according to an embodiment; and
FIG. 9 is a schematic diagram of a selection strategy of a three-lead electrocardiography monitoring method according to an embodiment.

## DESCRIPTION OF THE PREFERRED EMBODIMENTS

The disclosure will be further described in detail below through specific implementations in conjunction with the accompanying drawings. Associated similar element reference numerals are used for similar elements in different implementations. In the following implementations, many details are described such that the present application can be better understood. However, it may be effortlessly appreciated by a person skilled in the art that some of the features may be omitted, or may be substituted by other elements, materials, and methods in different cases. In certain cases, some operations involved in the present application are not displayed or described in the specification, which is to prevent a core part of the present application from being obscured by too much description. Moreover, for a person skilled in the art, the detailed description of the involved operations is not necessary, and the involved operations can be thoroughly understood according to the description in the specification and the general technical knowledge in the art.

In addition, the characteristics, operations, or features described in the specification can be combined in any appropriate manner to form various implementations. Moreover, the steps or actions in the method description can also be exchanged or adjusted in order in a way that is obvious to a person skilled in the art. Therefore, the various orders in the specification and the accompanying drawings are merely for the purpose of clear description of a certain embodiment and are not meant to be a necessary order unless otherwise stated that a certain order must be followed.

The serial numbers themselves for the components herein, for example, "first" and "second", are merely used to distinguish the described objects, and do not have any sequential or technical meaning. Moreover, as used in the present application, "connection" or "coupling", unless otherwise specified, comprises both direct and indirect connections (couplings).

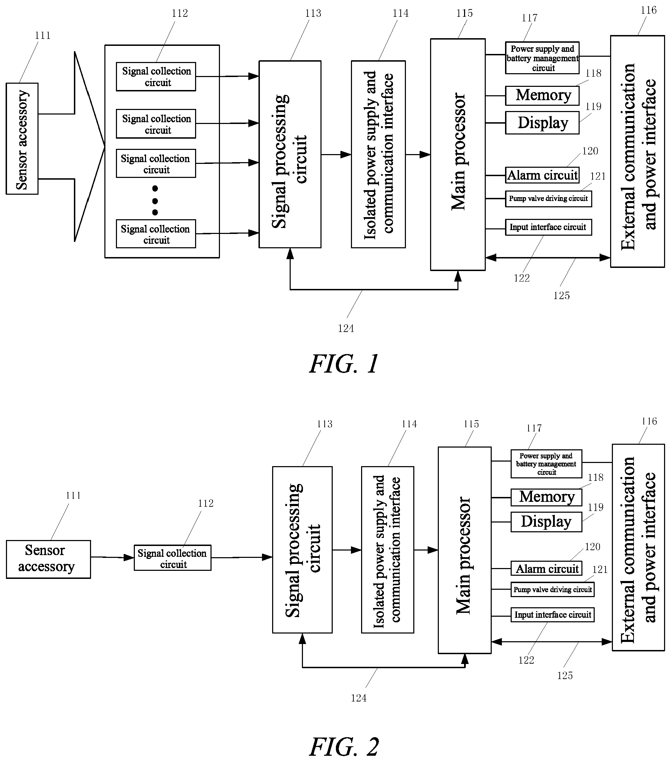

As shown in FIG. 1, a system framework diagram of a parameter processing module in a multi-parameter monitoring device is provided. The multi-parameter monitoring device may have an independent housing, and a sensor interface area may be arranged on a housing panel. A plurality of sensor interfaces may be integrated in the sensor interface area and configured to be connected to various external physiological parameter sensor accessories 111. The housing panel may further comprise a small IXD display area, a display 119, an input interface circuit 122, an alarm circuit 120 (such as an LED alarm area), and the like. The parameter processing module may have an external communication and power interface 116 for communicating with a host and obtaining power from the host. The parameter processing module may further support an externally inserted parameter module. The parameter module may be inserted to form a plug-in monitoring device host as a part of the monitoring device, or may be connected to the host by means of a cable, where the externally inserted parameter module may serve as an external accessory of the monitoring device.

An internal circuit of the parameter processing module may be placed in the housing. As shown in FIG. 1, the internal circuit may comprise signal collection circuits 112 corresponding to at least two physiological parameters, a front-end signal processor 113, and a main processor 115. The signal collection circuit 112 may be selected from an electrocardiogram circuit, a respiration circuit, a body temperature circuit, a blood oxygen circuit, a non-invasive blood pressure circuit, an invasive blood pressure circuit, and the like. These signal collection circuits 112 may be respectively electrically connected to corresponding sensor interfaces, so as to be electrically connected to sensor accessories 111 corresponding to different physiological parameters. An output end of the signal collection circuit may be coupled to the front-end signal processor 113, a communication port of the front-end signal processor 113 may be coupled to the main processor 115, and the main processor 115 may be electrically connected to the external communication and power interface 116. The sensor accessories 111 and the signal collection circuits 112 corresponding to various physiological parameters can use common circuits in the prior art. The front-end signal processor 113 may complete sampling and analog-to-digital conversion of an output signal of the signal collection circuit 112, and output a control signal to control a measurement process of a physiological signal. These parameters may comprise but are not limited to: parameters such as electrocardiogram, respiration, body temperature, blood oxygen, non-invasive blood pressure, and invasive blood pressure. The front-end signal processor 113 may be implemented by using a single-chip microcomputer or other semiconductor devices, for example, by using a single-chip microcomputer, an ASIC, or an FPGA. The front-end signal processor 113 may be powered by an isolated power supply, and data obtained through sampling may be sent to the main processor 115 through an isolated communication interface after being simply processed and packaged. For example, the front-end signal processor 113 may be coupled to the main processor 115 through an isolated power and communication interface 114. The reason why the front-end signal processor 113 is powered by the isolated power supply may be that the DC/DC power supply isolated by a transformer plays a role in isolating a patient from a power supply apparatus, with the main purposes of: 1. isolating the patient, and enabling an application part to be floating by means of an isolation transformer, so that a leakage current of the patient is small enough; and 2. preventing the voltage or energy during defibrillation or electrotome application from affecting a board card and a device of an intermediate circuit such as a main control board (guaranteed by a creepage distance and electrical clearance). Certainly, the front-end signal processor 113 may further be connected to the main processor 115 by means of a cable 124. The main processor 115 may complete calculation of the physiological parameter, and send a calculation result and waveforms of the parameter to the host (such as a host with a display, a PC, and a central station) through the external communication and power interface 116. The main processor 115 may be connected to the external communication and power interface 116 by means of a cable 125, to perform communication and/or obtain power. The parameter processing module may further comprise a power supply and battery management circuit 117. The power supply and battery management circuit 117 may obtain power from the host through the external communication and power interface 116, and supply power to the main processor 115 after processing such as rectification and filtering. The power supply and battery management circuit 117 may further monitor, manage, and protect the power obtained from the host through the external communication and power interface 116. The external communication and power interface 116 may be one of local area network interfaces composed of the Ethernet, a token ring, a token bus, and a fiber distributed data interface (FDDI) as the backbone network of these three networks, or a combination thereof, or may be one of wireless interfaces such as an infrared interface, a Bluetooth interface, a Wi-Fi interface, and a WMTS communication interface, or a combination thereof, or may be one of wired data connection interfaces such as an RS232 interface and a USB interface, or a combination thereof. The external communication and power interface 116 may also be one of a wireless data transmission interface and a wired data transmission interface or a combination thereof. The host may be any computer apparatus such as a host of a monitoring device, an electrocardiograph, an ultrasonic diagnostic apparatus, and a computer, and after being installed with matching software, the host can form a monitoring apparatus. The host may further be a communication apparatus such as a mobile phone, and the parameter processing module sends, by using a Bluetooth interface, data to the mobile phone supporting Bluetooth communication, so as to implement remote transmission of the data. After completing the calculation of the physiological parameter, the main processor 115 may further determine whether the physiological parameter is abnormal, and if yes, may give an alarm by means of the alarm circuit 120. A memory 118 may store intermediate and final data of the monitoring device, and store a program instruction or code executed by the main processor 115 and the like. If the monitoring device has a function of blood pressure measurement, the monitoring device may further comprise a pump and valve driving circuit 121. The pump and valve driving circuit 121 may be configured to perform inflation or deflation operations under the control of the main processor 115.

As shown in FIG. 2, a processing system architecture of a monitoring device with a single physiological parameter is provided. For the same content, reference may be made to the above content.

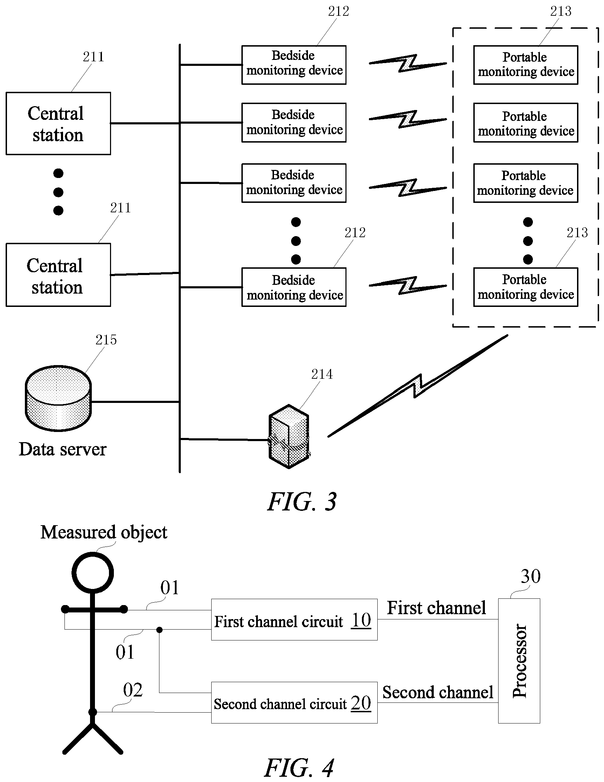

As shown in FIG. 3, a monitoring device networking system used in a hospital is provided. By using the system, data of the monitoring device may be saved as a whole to centrally manage patient information and nursing information that are stored in association, which facilitates storage of historical data and alarming in association. In the system shown in FIG. 3, a bedside monitoring device 212 may be provided for each hospital bed. The bedside monitoring device 212 may be the above multi-parameter monitoring device or a plug-in monitoring device. In addition, each bedside monitoring device 212 may further be paired with one portable monitoring apparatus 213 for transmission. The portable monitoring apparatus 213 provides a simple and portable parameter processing module, and the simple and portable parameter processing module may be worn on the body of a patient to perform mobile monitoring for the patient. After the portable monitoring apparatus 213 and the bedside monitoring device 212 perform wired or wireless communication, physiological data generated through mobile monitoring may be transmitted to the bedside monitoring device 212 for display, or transmitted, by using the bedside monitoring device 212, to a central station 211 for a doctor or a nurse to check, or transmitted to a data server 215 for storage by using the bedside monitoring device 212. In addition, the portable monitoring apparatus 213 may further directly transmit, by using a wireless network node 214 arranged in the hospital, the physiological data generated through mobile monitoring to the central station 211 for storage and display, or transmit, by using the wireless network node 214 arranged in the hospital, the physiological data generated through mobile monitoring to the data server 215 for storage. It can be seen that the data corresponding to the physiological parameter displayed on the bedside monitoring device 212 may originate from a sensor accessory directly connected to the monitor, or from the portable monitoring apparatus 213, or from the data server. The portable monitoring apparatus 213 may be connected to the sensor accessory 111 in a wired and/or wireless manner, and comprise a part or all of the circuits of the above parameter processing module. For example, isolation measures for isolating patients may not be provided in the portable monitoring apparatus 213 but provided outside the portable monitoring apparatus 213, for example, provided on the sensor accessory 111. The portable monitoring apparatus 213 may be equipped with a display screen for displaying a parameter calculation result and/or alarm prompt information. For example, the portable monitoring apparatus 213 may be a wireless sensor patch attached to the body, or a transfer monitor, or a telemetry apparatus.

According to the monitoring device and the networking system above, an example in which the signal collection circuit 112 is a three-lead electrocardiogram circuit is used, that is, an example in which the signal collection circuit 112 is a three-lead electrocardiography signal collection circuit is used. Because a weak electrocardiography signal is easily covered by the interference of power frequency in a collection process, a right leg drive circuit is usually introduced to the three-lead electrocardiography signal collection circuit. The right leg drive circuit averages voltages of two measurement-leads connected to a human body, and connects an average voltage obtained after inversion and amplification to the human body as a common-mode driving signal of the human body by means of a driving-lead. Therefore, when a differential voltage of the two measurement-leads is used as an EGC signal of one channel, a common-mode rejection ratio of the three-lead electrocardiography signal collection circuit can be improved, an amplitude of a common-mode interference signal in the collected ECG signal can be reduced, and the interference of power frequency may be greatly reduced, so that the weak electrocardiography signal will not be covered by the interference of power frequency. During research and practice, the inventor has found that since the above three-lead electrocardiography signal collection circuit can support only ECG waveform collection and analysis on one channel, there may be a relatively high probability of errors in the final electrocardiography parameter obtained through calculation and an electrocardiography result obtained through analysis.

In this embodiment of the disclosure, for the three-lead electrocardiography monitoring, the driving-lead is also used as a source of electrocardiography data, so that electrocardiography signal collection on two channels can be implemented through the three-lead electrocardiography monitoring. For example, a first channel for acquiring an electrocardiography signal is constructed by means of two measurement-leads, and a second channel for acquiring an electrocardiography signal is constructed by means of either of the measurement-leads and a driving-lead, thereby implementing electrocardiography signal collection on two channels under three-lead electrocardiography monitoring. By constructing two channels for collecting electrocardiography signals, the electrocardiography signal of either of the two channels may be corrected according to the electrocardiography signal of the other channel, or whether an electrocardiogram is normal may be determined through analysis in conjunction with the electrocardiography signals of the two channels.

In another aspect, in a process of actually collecting electrocardiography signals, a choice may be made based on situations to acquire one or both of the electrocardiography signal of the first channel and the electrocardiography signal of the second channel for processing. A choice may be made based on a magnitude of common-mode interference. Since the first channel is constructed by two measurement-leads, the second channel is constructed by either of the measurement-leads and the driving-lead, and the right leg drive circuit averages voltages of the two measurement-leads, and connects an average voltage obtained after inversion and amplification to the human body as a common-mode driving signal of the human body by means of the driving-lead, regardless of the magnitude of the common-mode interference itself, due to the existence of the right leg drive circuit, the common-mode interference usually has little influence on the first channel, but the second channel is easily affected by the common-mode interference. The magnitude of the common-mode interference may be measured by a common-mode interference amplitude. When the common-mode interference amplitude is relatively small, it may indicate that the common-mode interference is relatively small. In this case, although the second channel is easily affected by the common-mode interference, since the common-mode interference itself is relatively small, the actual common-mode interference to the second channel is relatively small. Therefore, a choice may be made to acquire the electrocardiography signal of the first channel and the electrocardiography signal of the second channel for processing. When the common-mode interference amplitude is relatively large, it indicates that the common-mode interference is relatively large. In this case, only the electrocardiography signal of the first channel may be selected for processing. In this case, the actual common-mode interference to the second channel may be relatively large, and if the electrocardiography signal of the second channel is still selected for processing, the final electrocardiography parameter obtained through calculation and the electrocardiography analysis result may be adversely affected.

Embodiment 1

Referring to FIG. 4, in an embodiment of a monitoring device, the monitoring device may be a three-lead electrocardiography monitoring device, which may comprise two measurement-lead electrodes 01 and one driving-lead electrode 03 that are attached to a measured object, a first channel circuit 10, a second channel circuit 20, and a processor 30. The sensor accessory 111 may be mainly composed of the two measurement-lead electrodes 01 and one driving-lead electrode 02. The signal collection circuit 112 may be mainly composed of the first channel circuit 10 and the second channel circuit 20. Moreover, the processor 30 may be implemented by the main processor 115 or the signal processor 113. Certainly, some functions of the processor 30 may be implemented by the main processor 115, and some functions are implemented by the signal processor 113. The following gives a detailed description of the three-lead electrocardiography monitoring device.

The two measurement-lead electrodes 01 and one driving-lead electrode 02 are configured to be attached to the measured object. For example, the two measurement-lead electrodes 01 may be respectively connected to a first intercostal space on a midclavicular line at the right border of the sternum at the right shoulder and a first intercostal space of a midclavicular line at the left border of the sternum of the measured object, and the driving-lead electrode 02 may be connected to a xiphoid process level on the left midclavicular line of the measured object.

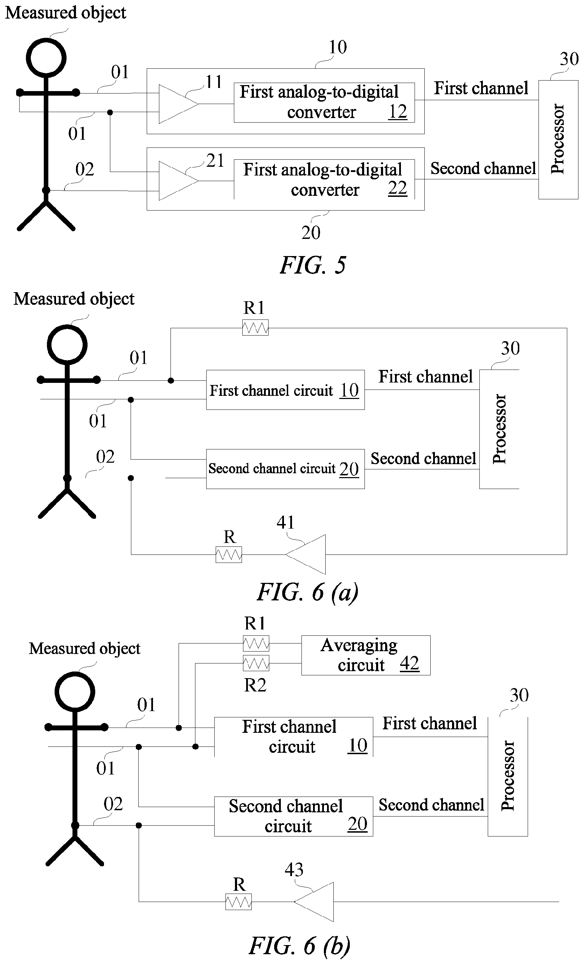

The first channel circuit 10 is connected to the two measurement-lead electrodes 01, and configured to acquire an electrocardiography signal of a first channel of the measured object by means of the two measurement-lead electrodes 01. Referring to FIG. 5, in an embodiment, the first channel circuit 10 may comprise a first differential amplifier 11 and a first analog-to-digital converter 12. Two input ends of the first differential amplifier 11 are respectively connected to the two measurement-lead electrodes 01, and may be configured to amplify a difference value between voltages of the two measurement-lead electrodes and output the amplified different value. An input end of the first analog-to-digital converter 12 is connected to an output end of the first differential amplifier 11, and may be configured to perform analog-to-digital conversion on the amplified difference value between the voltages, to acquire the electrocardiography signal of the first channel of the measured object.

The second channel circuit 20 is connected to either of the measurement-lead electrodes 01 and the driving-lead electrode 02, and may be configured to acquire an electrocardiography signal of a second channel of the measured object by means of the measurement-lead electrode 01 and the driving-lead electrode 02 that are connected to the second channel circuit. In an embodiment, the second channel circuit 20 may comprise a second differential amplifier 21 and a second analog-to-digital converter 22. Two input ends of the second differential amplifier 21 are respectively connected to either of the measurement-lead electrodes 01 and the driving-lead electrode 02, and configured to amplify a difference value between voltages of the measurement-lead electrode 01 and the driving-lead electrode 02 that are connected to the second differential amplifier, and output the amplified difference value. An input end of the second analog-to-digital converter 22 is connected to an output end of the second differential amplifier 21, and configured to perform analog-to-digital conversion on the amplified difference value between the voltages, to acquire the electrocardiography signal of the second channel of the measured object.

The processor 30 may choose to acquire one or both of the electrocardiography signal of the first channel and the electrocardiography signal of the second channel of the measured object for processing, to obtain an electrocardiography parameter of the measured object. A specific selection strategy of the processor 30 is described in detail below.

The processor 30 may make a choice based on a magnitude of common-mode interference. The magnitude of the common-mode interference may be measured by a common-mode interference amplitude, and there are many methods for acquiring the common-mode interference amplitude. For example, the processor 30 may acquire a common-mode interference amplitude in an electrocardiography signal of the measured object through one or both of the two channels (that is, the first channel and the second channel). Specifically, the processor 30 performs spectrum analysis on the electrocardiography signals/signal of the first channel and/or the second channel, and identifies an amplitude of a signal at a frequency of 50 Hz/60 Hz as the common-mode interference amplitude in the electrocardiography signal. Therefore, in an embodiment, the processor 30 may acquire the common-mode interference amplitude in the electrocardiography signal of the measured object by means of the two measurement-lead electrodes 01, and choose, according to the common-mode interference amplitude, to acquire one or both of the electrocardiography signal of the first channel and the electrocardiography signal of the second channel of the measured object for processing. Choosing, by the processor 30 according to the common-mode interference amplitude, to acquire one or both of the electrocardiography signal of the first channel and the electrocardiography signal of the second channel of the measured object for processing may comprise: choosing, based on a magnitude relationship between the common-mode interference amplitude and a threshold, to acquire one or both of the electrocardiography signal of the first channel and the electrocardiography signal of the second channel of the measured object for processing.

In an embodiment, choosing, by the processor 30 to acquire one or both of the electrocardiography signal of the first channel and the electrocardiography signal of the second channel of the measured object for processing may comprise: choosing to acquire the electrocardiography signal of the first channel of the measured object for processing, or choosing to acquire the electrocardiography signal of the first channel and the electrocardiography signal of the second channel of the measured object for processing. Since the common-mode interference usually has little influence on the first channel, both the choice made to acquire the electrocardiography signal of the first channel of the measured object for processing and the choice made to acquire the electrocardiography signal of the first channel and the electrocardiography signal of the second channel of the measured object for processing ensure that at least the electrocardiography signal of the first channel is selected for processing. Since the processor 30 makes a choice based on the magnitude of the common-mode interference, when determining that the common-mode interference amplitude is greater than or equal to the threshold, the processor 30 may choose to acquire only the electrocardiography signal of the first channel of the measured object for processing instead of choosing to acquire the electrocardiography signal of the second channel with a low common-mode rejection ratio for processing, thereby improving accuracy of a calculated electrocardiography parameter and an electrocardiography analysis result when the common-mode interference is relatively large. When determining that the common-mode interference amplitude is less than the threshold, the processor chooses to acquire the electrocardiography signal of the first channel and the electrocardiography signal of the second channel of the measured object for processing. Compared with conventional three-lead monitoring during which an electrocardiography signal of only one channel can be collected for processing, collecting electrocardiography signals of two channels for processing can provide the accuracy of the calculated electrocardiography parameter and the electrocardiography analysis result.

When the processor 30 chooses to acquire only the electrocardiography signal of the first channel of the measured object for processing, one or two of the following items may be comprised.

(1.1) Generating, according to the electrocardiography signal of the first channel of the measured object, an electrocardiogram waveform for displaying.

(1.2) Determining, according to the electrocardiography signal of the first channel of the measured object through analysis, whether an electrocardiogram of the measured object is normal.

When the processor 30 chooses to acquire the electrocardiography signal of the first channel and the electrocardiography signal of the second channel of the measured object for processing, one or more of the following four items may be comprised.

(2.1) Generating, according to the electrocardiography signal of the first channel of the measured object, an electrocardiogram waveform for displaying.

(2.2) Generating, according to the electrocardiography signal of the second channel of the measured object, an electrocardiogram waveform for displaying.

(2.3) Correcting the electrocardiography signal of either of the channels of the measured object based on the electrocardiography signal of the other channel. For example, when one of the electrocardiography signals of the two channels has loud noise and the other has almost no noise, the electrocardiography signal with almost no noise may be used to correct the electrocardiography signal with loud noise. For another example, when one electrocardiography signal of the two channels has loud noise at a time period T1 but has almost no noise at a time period T2, and the other electrocardiography signal has almost no noise at the time period T1 but has loud noise at the time period T2, the electrocardiography signal that has almost no noise at the time period T1 may be used to correct the electrocardiography signal that has loud noise at the time period T1, and the electrocardiography signal that has almost

no noise at the time period T2 is used to correct the electrocardiography signal that has loud noise at the time period T2.

(2.4) determining, according to the electrocardiography signal of the first channel and the electrocardiography signal of the second channel of the measured object through analysis, whether an electrocardiogram of the measured object is normal. For example, a PVC characteristic in an electrocardiography signal of one channel is not obvious, and a PVC characteristic in an electrocardiography signal of the other channel is relatively obvious, so that it may be determined, in conjunction with the electrocardiography signals of the two channels, that the measured object has the phenomenon of premature ventricular bigeminy.

Compared with the conventional three-lead electrocardiography monitoring that supports only electrocardiography signal collection on one channel, if the electrocardiography signal of this channel has loud noise, it is basically impossible to analyze the electrocardiography signal to obtain an electrocardiography parameter and an electrocardiography result. However, the disclosure can implement electrocardiography signal collection on two channels. When an electrocardiography signal of one channel has loud noise, if an electrocardiography signal of the other channel has low noise in this case, the electrocardiography signal may further be analyzed to obtain a relatively accurate electrocardiography parameter and electrocardiography result.

It should be noted that, for the electrocardiography signal of either of the channels, when the processor 30 does not choose to acquire the electrocardiography signal of the channel, there are many implementation methods. For example, in a first implementation method, the processor 30 shields the electrocardiography signal of the channel, that is, although the processor 30 can receive the electrocardiography signal of the channel, the electrocardiography signal of the channel will not be processed. For example, in a second implementation method, the second channel is used as an example. Since the second channel circuit 20 is connected to one measurement-lead electrode 01 and the driving-lead electrode 02, a switch component controlled by the processor 30 may be introduced. The switch component is located on a line of the second channel circuit 20 and the measurement-lead electrode 01 connected to the second channel circuit, or on a line connecting the second channel circuit 20 to the driving-lead electrode 02, or on a line connecting the second channel circuit 20 to the processor 30, so that when the processor 30 controls the switch component to be non-conductive, the processor 30 cannot receive the electrocardiography signal of the second channel. For the first channel, a switch component controlled by the processor 30 may also be introduced in a similar manner. Details are not described herein again.

The three-lead electrocardiography monitoring device in one embodiment may display the generated electrocardiogram waveform by means of the display 119. For example, when the processor 30 chooses to acquire only the electrocardiography signal of the first channel of the measured object for processing, the display 119 displays only an electrocardiogram waveform of a single channel (that is, the first channel). When the processor 30 chooses to acquire the electrocardiography signal of the first channel and the electrocardiography signal of the second channel of the measured object for processing, the display 119 may display electrocardiogram waveforms of two channels (that is, the first channel and the second channel). Therefore, when the processor performs, based on situations, switching between choosing only the electrocardiography signal of one channel for processing and choosing electrocardiography signals of two channels for processing, the display 119 also switches between displaying the electrocardiogram waveform of the single electrocardiography signal channel and displaying the electrocardiogram waveforms of the two channels.

In order to reduce the common-mode interference, the three-lead electrocardiography monitoring device may comprise a right leg drive circuit. A source of a right leg drive signal of the right leg drive circuit may be a signal obtained after a voltage of either of the measurement-lead electrodes 01 is inverted and amplified, or may be a signal obtained after voltages of the two measurement-lead electrodes 01 are averaged and an average voltage is inverted and amplified, or may even be a constant voltage signal. Details are described below.

For example, in a first embodiment of the right leg drive circuit, referring to FIG. 6 (a), the right leg drive circuit may comprise an inverting amplifier 41 connected to either of the measurement-lead electrodes 01 and configured to provide, to the driving-lead electrode 02 as a first right leg drive signal, a voltage of the connected measurement-lead electrode 01 that is obtained after inversion and amplification.

For example, in a second embodiment of the right leg drive circuit, referring to FIG. 6 (b), the right leg drive circuit may comprise an averaging circuit 42 and an inverting amplifier 43. Two input ends of the averaging circuit 42 are respectively connected to the two measurement-lead electrodes 01, and configured to output an average voltage of the two measurement-lead electrodes to the inverting amplifier 43. The inverting amplifier 43 is configured to provide the average voltage obtained after inversion and amplification to the driving-lead electrode 02 as the first right leg drive signal.

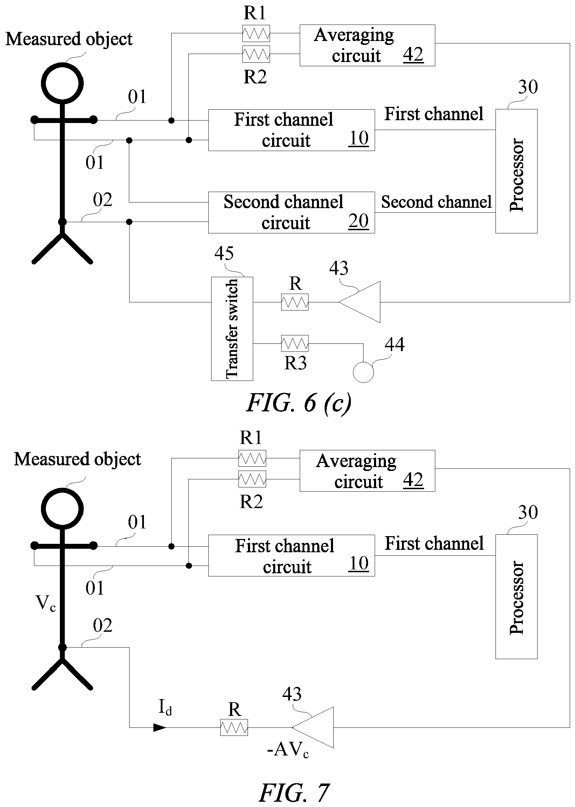

On the basis of the first embodiment or the second embodiment of the right leg drive circuit, referring to FIG. 6 (c), the right leg drive circuit may comprise a constant voltage source 44 for providing a constant voltage signal, and the constant voltage source is connected to the driving-lead electrode 02 and configured to provide the constant voltage signal to the driving-lead electrode 02 as a second right leg drive signal. In addition, the right leg drive circuit may further comprise a switch 45, and the switch 45 may be implemented by using an analog switch or a digital relay. When the common-mode interference amplitude is relatively small (for example, when the common-mode interference amplitude is less than the threshold), the processor 30 provides the second right leg drive signal to the driving-lead electrode 02 after switching performed by means of the switch 45, or still provides the first right leg drive signal to the driving-lead electrode 02. Alternatively, when the processor 30 chooses to acquire the electrocardiography signal of the first channel and the electrocardiography signal of the second channel of the measured object for processing, the processor 30 provides the second right leg drive signal to the driving-lead electrode instead 02 after switching performed by means of the switch 45, or still provides the first right leg drive signal to the driving-lead electrode 02.

It should be noted that FIG. 6 (c) is a schematic structural diagram in which a constant voltage source 44 is introduced based on the second embodiment of the right leg drive circuit. In addition, some resistors may further be arranged as required in each of the right leg drive circuits in FIG. 6, for example, resistors R1, R2, R3, and R shown in the figure.

The right leg drive circuit is analyzed below. The right leg drive circuit shown in FIG. 6 (b) is used as an example. When the processor 30 chooses to acquire only the electrocardiography signal of the first channel of the measured object for processing, the circuit in FIG. 6 (b) may be equivalent to the circuit shown in FIG. 7. In this case, if a common-mode interference amplitude of a human body is $V_c$, an amplification factor of the inverting amplifier 43 is -A, and a leakage current passing through the human body is $I_d$, the following is obtained:

$$V_c = RI_d - AV_c \,;$$

thus, the following may be calculated:

$$V_c = \frac{RI_d}{1+A} \,.$$

Therefore, a parameter A may be adjusted by selecting an actual device of the inverting amplifier 43. When the parameter A is relatively large, the common-mode interference amplitude $V_c$ is relatively small, that is, the common-mode interference amplitude entering a channel (for example, the first channel) in which a signal is used is greatly reduced.

The three-lead electrocardiography monitoring device in any one of the above embodiments may be a mobile monitoring device, for example, a wearable monitoring device, and a user may acquire monitoring situations from a display of the wearable monitoring device.

It should be noted that the three-lead electrocardiography monitoring device disclosed in FIG. 4 to FIG. 7 is also a type of monitor. Therefore, for some other structures and components of the three-lead electrocardiography monitoring device disclosed in FIG. 4 to FIG. 7, reference may also be made to the monitors disclosed in FIG. 1 and FIG. 2. For example, the processor 30 in the monitor in FIG. 4 to FIG. 7 may be implemented by the main processor 115 or the signal processor 113 in FIG. 1 and FIG. 2, or some functions of the processor 30 are implemented by the main processor 115, and some functions are implemented by the signal processor 113. The two measurement-lead electrodes 01 and one driving-lead electrode 02 in the monitor in FIG. 4 to FIG. 7 may be implemented by the sensor accessory 111 in FIG. 1 and FIG. 2. In addition, the three-lead electrocardiography monitoring device disclosed in FIG. 4 to FIG. 7 may further comprise other components disclosed in FIG. 1 and FIG. 2, for example, the isolated power supply and communication interface 114 and the external communication and power interface 116, which are not described in detail herein. Furthermore, the three-lead electrocardiography monitoring device disclosed in FIG. 4 to FIG. 7 may also be all or part of the portable monitoring apparatus 213 shown in FIG. 3. For example, some of the functions of the processor 30 in FIG. 4 to FIG. 7 may be performed in the portable monitoring apparatus 213, and some of the functions may be performed in the bedside monitor 212, or all the functions of the processor 30 are performed in the portable monitoring apparatus 213, and processed data is displayed, output, and stored by means of the central station 211 or the bedside monitor 212.

Embodiment 2

An embodiment further provides a three-lead electrocardiography monitoring method, which can be applied to the monitoring device disclosed above. Three leads comprise two measurement-lead electrodes 01 and one driving-lead electrode 02 that are configured to be attached to a measured object. According to the three-lead electrocardiography monitoring method of the disclosure, two channels for collecting electrocardiography signals are constructed by means of the three leads. Detailed description is given below.

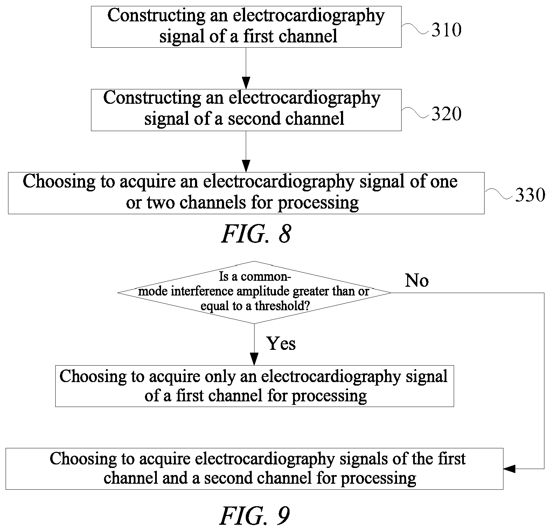

Referring to FIG. 8, in an embodiment of the three-lead electrocardiography monitoring method, the method may comprise steps 310 to 330.

Step 310, constructing an electrocardiography signal of a first channel. For example, the electrocardiography signal of the first channel of the measured object is acquired by means of the two measurement-lead electrodes.

Step 320, constructing an electrocardiography signal of a second channel. For example, the electrocardiography signal of the second channel of the measured object is acquired by means of either of the measurement-lead electrodes and the driving-lead electrode.

Step 330, choosing to acquire the electrocardiography signal of one or two channels for processing. Specifically, a choice is made to acquire one or both of the electrocardiography signal of the first channel and the electrocardiography signal of the second channel of the measured object for processing, to obtain an electrocardiography parameter of the measured object.

A selection strategy of step 330 is specifically described below.

In step 330, a choice may be made based on a magnitude of common-mode interference. The magnitude of the common-mode interference may be measured by a common-mode interference amplitude, and there are many methods for acquiring the common-mode interference amplitude. For example, a common-mode interference amplitude in an electrocardiography signal of the measured object may be acquired through one or both of the two channels (that is, the first channel and the second channel). Specifically, the processor 30 performs spectrum analysis on the electrocardiography signals/signal of the first channel and/or the second channel, and identifies an amplitude of a signal at a frequency of 50 Hz/60 Hz as the common-mode interference amplitude in the electrocardiography signal. Therefore, in an embodiment, in step 330, the common-mode interference amplitude in the electrocardiography signal of the measured object may be acquired by means of the two measurement-lead electrodes 01, and a choice is made, according to the common-mode interference amplitude, to acquire one or both of the electrocardiography signal of the first channel and the electrocardiography signal of the second channel of the measured object for processing. In step 330, choosing, according to the common-mode interference amplitude, to acquire one or both of the electrocardiography signal of the first channel and the electrocardiography signal of the second channel of the measured object for processing may comprise: choosing, based on a magnitude relationship between the common-mode interference amplitude and a threshold, to acquire one or both of the electrocardiography signal of the first channel and the electrocardiography signal of the second channel of the measured object for processing.

In an embodiment, in step 330, choosing to acquire one or both of the electrocardiography signal of the first channel and the electrocardiography signal of the second channel of the measured object for processing may comprise: choosing to acquire the electrocardiography signal of the first channel of the measured object for processing, or choosing to acquire the electrocardiography signal of the first channel and the electrocardiography signal of the second channel of the measured object for processing. Since the common-mode interference usually has little influence on the first channel, both the choice made to acquire the electrocardiography signal of the first channel of the measured object for processing and the choice made to acquire the electrocardiography signal of the first channel and the electrocardiography signal of the second channel of the measured object for processing ensure that at least the electrocardiography signal of the first channel is selected for processing. Referring to FIG. 9, since a choice is made based on the magnitude of the common-mode interference in step 330, in step 330, when it is determined that the common-mode interference amplitude is greater than or equal to the threshold, a choice is made to acquire only the electrocardiography signal of the first channel of the measured object for processing instead of acquiring the electrocardiography signal of the second channel with a low common-mode rejection ratio for processing, thereby improving accuracy of a calculated electrocardiography parameter and an electrocardiography analysis result when the common-mode interference is relatively large. When it is determined that the common-mode interference amplitude is less than the threshold, a choice is made to acquire the electrocardiography signal of the first channel and the electrocardiography signal of the second channel of the measured object for processing. Compared with conventional three-lead monitoring during which an electrocardiography signal of only one channel can be collected for processing, collecting electrocardiography signals of two channels for processing can provide the accuracy of the calculated electrocardiography parameter and the electrocardiography analysis result.

When a choice is made to acquire only the electrocardiography signal of the first channel of the measured object for processing in step 330, one or two of the following items may be comprised.

(1.1) Generating, according to the electrocardiography signal of the first channel of the measured object, an electrocardiogram waveform for displaying.

(1.2) Determining, according to the electrocardiography signal of the first channel of the measured object through analysis, whether an electrocardiogram of the measured object is normal.

When a choice is made to acquire the electrocardiography signal of the first channel and the electrocardiography signal of the second channel of the measured object for processing in step 330, one or more of the following four items may be comprised.

(2.1) Generating, according to the electrocardiography signal of the first channel of the measured object, an electrocardiogram waveform for displaying.

(2.2) Generating, according to the electrocardiography signal of the second channel of the measured object, an electrocardiogram waveform for displaying.

(2.3) Correcting the electrocardiography signal of either of the channels of the measured object based on the electrocardiography signal of the other channel.

(2.4) determining, according to the electrocardiography signal of the first channel and the electrocardiography signal of the second channel of the measured object through analysis, whether an electrocardiogram of the measured object is normal.

In order to reduce the common-mode interference, in an embodiment of the three-lead electrocardiography monitoring method, a right leg drive signal may further be introduced. A source of the right leg drive signal may be a signal obtained after a voltage of either of the measurement-lead electrodes 01 is inverted and amplified, or may be a signal obtained after voltages of the two measurement-lead electrodes 01 are averaged and an average voltage is inverted and amplified, or may even be a constant voltage signal. For example, in an embodiment, the voltages of the two measurement-lead electrodes are averaged, and an average voltage is provided to the driving-lead electrode as a first right leg drive signal after being inverted and amplified. Alternatively, a voltage of either of the measurement-lead electrodes is provided to the driving-lead electrode as the first right leg drive signal after being inverted and amplified. For another example, in an embodiment, the constant voltage signal is provided to the driving-lead electrode as a second right leg drive signal. The first right leg drive signal is provided to the driving-lead electrode by default, and when a choice is made to acquire the electrocardiography signal of the first channel and the electrocardiography signal of the second channel of the measured object for processing, the second right leg drive signal is provided to the driving-lead electrode after switching, or the first right leg drive signal is still provided to the driving-lead electrode.

A person skilled in the art may understand that all or some of the functions of the various methods in the above implementations may be implemented by means of hardware or by means of a computer program. When all or some of the functions in the above implementations are implemented by means of a computer program, the program may be stored in a computer-readable storage medium, and the storage medium may comprise: a read-only memory, a random access memory, a magnetic disk, an optical disk, a hard disk, and the like, and the program is executed by a computer to implement the above functions. For example, the program is stored in a memory of an apparatus, and when the program in the memory is executed by means of a processor, all or some of the above functions can be implemented. In addition, when all or some of the functions in the above implementations are implemented by means of a computer program, the program may also be stored in a storage medium such as a server, another computer, a magnetic disk, an optical disk, a flash disk, or a mobile hard disk, may be saved in a memory of a local apparatus through downloading or copying, or version updating may be performed on a system of the local apparatus. When the program in the memory is executed by means of a processor, all or some of the functions in the above implementations can be implemented.

The disclosure has been described by using specific examples above, which are merely for the purpose of facilitating understanding of the disclosure and are not intended to limit the disclosure. For a person of ordinary skill in the art, changes may be made to the above specific implementations according to the idea of the disclosure.

**Claims**

1. A three-lead electrocardiography monitoring method, the three leads comprising two measurement-lead electrodes and one driving-lead electrode that are attached to a measurement object, the method comprises:

   acquiring an electrocardiography signal of a first channel of the measured object by means of the two measurement-lead electrodes; and acquiring an electrocardiography signal of a second channel of the measured object by means of the driving-lead electrode and either of the two measurement-lead electrodes; and
   **characterized in that** the method further comprises:
   acquiring a common-mode interference amplitude in an electrocardiography signal of the measured object by means of one or both of the two channels; and choosing, according to the common-mode interference amplitude, one or both of the acquired electrocardiography signal of the first channel and the electrocardiography signal of the second channel for processing, to obtain an electrocardiography parameter of the measured object.

2. The three-lead electrocardiography monitoring method of claim 1, wherein when the common-mode interference amplitude is less than a threshold, a choice is made to acquire the electrocardiography signal of the first channel and the electrocardiography signal of the second channel for processing.

3. The three-lead electrocardiography monitoring method of claim 1, wherein when it is determined that the common-mode interference amplitude is greater than or equal to a threshold, a choice is made to acquire only the electrocardiography signal of the first channel for processing.

4. The three-lead electrocardiography monitoring method of claim 1, wherein voltages of the two measurement-lead electrodes are averaged, and an average voltage is provided to the driving-lead electrode as a first right leg drive signal after being inverted and amplified; or a voltage of either of the two measurement-lead electrodes is provided to the driving-lead electrode as the first right leg drive signal after being inverted and amplified.

5. A three-lead electrocardiography monitoring device, comprising:

two measurement lead electrodes (01) and one driving lead electrode (02) that are attached to a measured object;
a first channel circuit (10) connected to the two measurement lead electrodes (01) and configured to acquire an electrocardiography signal of a first channel of the measured object by means of the two measurement lead electrodes (01);
a second channel circuit (20) connected to either of the measurement lead electrodes (01) and the driving lead electrode (02), and configured to acquire an electrocardiography signal of a second channel of the measured object by means of the measurement lead electrode (01) and the driving lead electrode (02) that are connected to the second channel circuit (20); and
a processor (30); **characterized in that** the processor (30) is configured to acquire a common-mode interference amplitude in an electrocardiography signal of the measured object by means of one or both of the two channels, and choose, according to the common-mode interference amplitude, one or both of the acquired electrocardiography signal of the first channel and the electrocardiography signal of the second channel of the measured object for processing, to obtain an electrocardiography parameter of the measured object.

6. The three-lead electrocardiography monitoring device of claim 5, wherein when it is determined that the common-mode interference amplitude is less than a threshold, the processor (30) chooses to acquire the electrocardiography signal of the first channel and the electrocardiography signal of the second channel for processing.

7. The three-lead electrocardiography monitoring device of claim 6, wherein the processor (30) chooses to acquire the electrocardiography signal of the first channel and the electrocardiography signal of the second channel for processing comprises:
the processor (30) is further configured to:

generate, according to the electrocardiography signal of the first channel of the measured object, an electrocardiogram waveform for displaying; and/or
generate, according to the electrocardiography signal of the second channel of the measured object, an electrocardiogram waveform for displaying; and/or
correct the electrocardiography signal of either of the channels of the measured object based on the electrocardiography signal of the other channel; and/or
determine, according to the electrocardiography signal of the first channel and the electrocardiography signal of the second channel through analysis, whether an electrocardiogram of the measured object is normal.

8. The three-lead electrocardiography monitoring device of claim 5, wherein when it is determined that the common-mode interference amplitude is greater than or equal to a threshold, the processor (30) chooses to acquire only the electrocardiography signal of the first channel for processing.

9. The three-lead electrocardiography monitoring device of claim 8, wherein the processor (30) chooses to acquire only the electrocardiography signal of the first channel for processing comprises:
the processor (30) is further configured to:

generate, according to the electrocardiography signal of the first channel of the measured object, an electrocardiogram waveform for displaying; and/or
determine, according to the electrocardiography signal of the first channel through analysis, whether an electrocardiogram of the measured object is normal.

10. The three-lead electrocardiography monitoring device of claim 5, further comprising:

a first inverting amplifier (41) connected to either of the measurement-lead electrodes (01) and configured to provide a voltage of the connected measurement-lead electrode (01) that is obtained after inversion and amplification to the driving-lead electrode (02) as a first right leg drive signal; or

an averaging circuit (42) and a second inverting amplifier (43), wherein two input ends of the averaging circuit (42) are respectively connected to the two measurement-lead electrodes (01), and are configured to output an average voltage of the two measurement-lead electrodes (01) to the second inverting amplifier (43); and the second inverting amplifier (43) is configured to provide the average voltage obtained after inversion and amplification to the driving-lead electrode (02) as a first right leg drive signal.

11. The three-lead electrocardiography monitoring device of claim 10, further comprising:

a constant voltage source (44) connected to the driving-lead electrode (02), and configured to provide a constant voltage signal to the driving-lead electrode as a second right leg drive signal; and

a switch, wherein when the processor chooses to acquire the electrocardiography signal of the first channel and the electrocardiography signal of the second channel of the measured object for processing, the processor provides the second right leg drive signal to the driving-lead electrode after switching performed by means of the switch, or still provides the first right leg drive signal to the driving-lead electrode.

12. The three-lead electrocardiography monitoring device of claim 5, wherein the first channel circuit comprises:

a first differential amplifier, two input ends of the first differential amplifier are respectively connected to the two measurement-lead electrodes, and configured to amplify a difference value between voltages of the two measurement-lead electrodes and output the amplified different value; and

a first analog-to-digital converter, an input end of the first analog-to-digital converter is connected to an output end of the first differential amplifier, and configured to perform analog-to-digital conversion on the amplified difference value between the voltages, and acquire the electrocardiography signal of the first channel of the measured object.

13. The three-lead electrocardiography monitoring device of claim 5, wherein the second channel circuit comprises:

a second differential amplifier, two input ends of the second differential amplifier are respectively connected to the driving-lead electrode and either of the two measurement-lead electrodes, and configured to amplify a difference value between voltages of the measurement-lead electrode and the driving-lead electrode that are connected to the second differential amplifier, and output the amplified difference value; and

a second analog-to-digital converter, an input end of the second analog-to-digital converter is connected to an output end of the second differential amplifier, and configured to perform analog-to-digital conversion on the amplified difference value between the voltages, and acquire the electrocardiography signal of the second channel of the measured object.

**Patentansprüche**

1. Verfahren zur Überwachung des Elektrokardiogramms mit drei Kanälen, die zwei Messleitungselektroden und eine Antriebsleitungselektrode umfassen, die an einem Messobjekt befestigt ist, wobei das Verfahren Folgendes beinhaltet:

Erfassen eines EKG-Signals eines ersten Kanals des Messobjekts mittels der beiden Messleitungselektroden; und Erfassen eines EKG-Signals eines zweiten Kanals des Messobjekts mittels der Antriebsleitungselektrode und einer der beiden Messleitungselektroden; und dadurchgekennzeichnet, dass das Verfahren ferner Folgendes umfasst:
Erfassen einer Amplitude der Gleichtaktstörung in einem EKG-Signal des Messobjekts mittels eines der beiden Kanäle oder beider Kanäle; und Auswählen des erfassten EKG-Signals des ersten Kanals oder des EKG-Signals des zweiten Kanals entsprechend der Amplitude der Gleichtaktstörung zur Verarbeitung, um einen EKG-Parameter des Messobjekts zu erhalten.

2. Verfahren zur Überwachung des Elektrokardiogramms mit drei Kanälen nach Anspruch 1, bei dem, wenn die Amplitude der Gleichtaktstörung kleiner als ein Schwellenwert ist, entschieden wird, das EKG-Signal des ersten Kanals und das EKG-Signal des zweiten Kanals zur Verarbeitung zu erfassen.

3. Verfahren zur Überwachung des Elektrokardiogramms mit drei Kanälen nach Anspruch 1, bei dem, wenn die Amplitude der Gleichtaktstörung größer oder gleich dem Schwellenwert ist, entschieden wird, nur das EKG-Signal des ersten Kanals zur Verarbeitung zu erfassen.

4. Verfahren zur Überwachung des Elektrokardiogramms mit drei Kanälen nach Anspruch 1, bei dem die Spannungen der beiden Messleitungselektroden gemittelt werden und eine Durchschnittsspannung an die Antriebsleitungselektrode als ein erstes Ansteuerungssignal für das rechte Bein bereitgestellt wird, nachdem es invertiert und verstärkt wurde; oder eine Spannung von einem der beiden Messleitungselektroden nach Invertierung und Verstärkung als erstes Ansteuersignal für das rechte Bein an die Antriebsleitungselektrode bereitgestellt wird.

5. Verfahren zur Überwachung des Elektrokardiogramms mit drei Kanälen, das Folgendes umfasst:

   zwei Messleitungselektroden (01) und eine Antriebsleitungselektrode (02), die an einem Messobjekt angebracht sind; eine erste Kanalschaltung (10), die mit den beiden Messleitungselektroden (01) verbunden ist und ausgelegt ist, um ein EKG-Signal eines ersten Kanals des Messobjekts mit Hilfe der beiden Messleitungselektroden (01) zu erfassen; eine zweite Kanalschaltung (20), die mit einer der Messleitungselektroden (01) und der Antriebsleitungselektrode (02) verbunden ist und ausgelegt ist, um ein EKG-Signal eines zweiten Kanals des Messobjekts mittels der Messleitungselektrode (01) und der Antriebsleitungselektrode (02) zu erfassen, die mit der zweiten Kanalschaltung (20) verbunden sind; und einen Prozessor (30), der so ausgelegt ist, dass er eine Amplitude der Gleichtaktstörung in einem EKG-Signal des Messobjekts, mittels eines der beiden oder beider Kanäle erfasst und entsprechend der Amplitude der Gleichtaktstörung eines oder beide des erfassten EKG-Signals des ersten Kanals und des EKG-Signals des zweiten Kanals des Messobjekts zur Verarbeitung auswählt, um einen EKG-Parameter des Messobjekts zu erhalten.

6. Verfahren zur Überwachung des Elektrokardiogramms mit drei Kanälen nach Anspruch 5, bei dem, wenn die Amplitude der Gleichtaktstörung kleiner ist als ein Schwellenwert, der Prozessor (30) entscheidet, das EKG-Signal des ersten Kanals und das EKG-Sgnal des zweiten Kanals für die Verarbeitung zu erfassen

7. Verfahren zur Überwachung des Elektrokardiogramms mit drei Kanälen nach Anspruch 6, wobei der Prozessor (30) die Erfassung des EKG-Signals des ersten Kanals und des EKG-Signals des zweiten Kanals zur Verarbeitung entscheidet, und Folgendes beinhaltet:
der Prozessor (30) ist ferner so konfiguriert, dass er:

   entsprechend dem EKG-Signal des ersten Kanals des Messobjekts, eine EKG Wellenform zur Anzeige zu erzeugen; und/oder entsprechend dem EKG-Signal des zweiten Kanals des Messobjekts eine EKG-Wellenform zur Anzeige zu erzeugen; und/oder das EKG-Signal eines der Kanäle des Messobjekts entsprechend des EKG-Signals des anderen Kanals zu korrigieren; und/oder anhand des EKG-Signals des ersten Kanals und des EKG-Signals des zweiten Kanals durch Analyse bestimmen, ob ein Elektrokardiogramm des Messobjekts normal ist.

8. Verfahren zur Überwachung des Elektrokardiogramms mit drei Kanälen nach Anspruch 5, wobei, wenn die Amplitude der Gleichtaktstörung größer oder gleich einem Schwellenwert ist, der Prozessor (30) entscheidet, nur das EKG-Signal des ersten Kanals zur Verarbeitung zu erfassen.

9. Verfahren zur Überwachung des Elektrokardiogramms mit drei Kanälennach Anspruch 8, wobeider Prozessor (30) beschließt, nur das EKG-Signal des ersten Kanals zur Verarbeitung zu erfassen, und Folgendes beinhaltet:
der Prozessor (30) ist ferner so konfiguriert, dass er:

   entsprechend dem EKG-Signal des ersten Kanals des Messobjekts eine EKG Wellenform zur Anzeige zu erzeugen; und/oder entsprechend dem EKG-Signal des ersten Kanals durch Analyse zu bestimmen, ob ein Elektrokardiogramm des Messobjekts normal ist.

10. Verfahren zur Überwachung des Elektrokardiogramms mit drei Kanälen nach Anspruch 5, das ferner Folgendes umfassend:

   einen ersten invertierenden Verstärker (41), der mit einer der beiden Messleitungselektroden (01) verbunden und so ausgelegt ist, dass er eine Spannung der angeschlossenen Messleitungselektrode (01), die nach Invertierung und Verstärkung erhalten wird, an die Antriebsleitungselektrode (02) als ein erstes Ansteuersignal für das rechte Bein bereitstellt; oder eine gemittelte Schaltung (42) und einen zweiten invertierenden Verstärker (43), wobei die beiden Eingangsenden der gemittelten Schaltung (42) jeweils mit den beidenMessleitungselektroden (01) verbunden und so ausgelegt sind, dass sie eine Durchschnittsspannung der beiden Messleitungselektroden (01) an den zweiten invertierenden Verstärker (43) ausgeben; und der zweite invertierende Verstärker (43) ist so konfiguriert, dass er die nach der Invertierung und Verstärkung erhaltene Durchschnittsspannung an die Antriebsleitungselektrode (02) als ein erstes Ansteuerungssignal für das rechte Bein bereitstellt.

11. Verfahren zur Überwachung des Elektrokardiogramms mit drei Kanälennach Anspruch 10, das ferner Folgendes umfassend:

   eine Konstantspannungsquelle (44), die mit der Antriebsleitungselektrode (02) verbunden und so konfiguriert ist, dass sie ein Konstantspannungssignal an die Antriebsleitungselektrode als zweites Ansteuerungssignal für das rechte Bein bereitstellt; und
   einen Schalter, wobei der Prozessor, wenn er entscheidet, das EKG-Signal des ersten Kanals und das EKG-Signal des zweiten Kanals des Messobjekts zur Verarbeitung zu erfassen, nach dem Umschalten das zweite Ansteuerungssignal des rechten Beins an die Antriebsleitungselektrode bereitstellt, oder noch das erste Ansteuerungssignal des rechten Beins an die Antriebsleitungselektrode liefert.
   einen Schalter, wobei der Prozessor, wenn er entscheidet, das EKG-Signal des ersten Kanals und das EKG-Signal des zweiten Kanals des Messobjekts zur Verarbeitung zu erfassen, nach dem Umschalten das zweite Ansteuerungssignal des rechten Beins an die Antriebsleitungselektrode bereitstellt, oder noch das erste Ansteuerungssignal des rechten Beins an die Antriebsleitungselektrode liefert.

12. Verfahren zur Überwachung des Elektrokardiogramms mit drei Kanälennach Anspruch 5, wobei die erste Kanalschaltung Folgendes umfasst:

   einen ersten Differenzverstärker, wobei zwei Eingangsenden des ersten Differenzverstärkers jeweils mit den beiden Messleitungselektroden verbunden sind und so ausgelegt sind, dass sie einen Differenzwert zwischen den Spannungen der beiden Messleitungselektroden verstärken und den verstärkten Differenzwert ausgeben; und
   einen ersten Analog-Digital-Wandler, wobei ein Eingangsende des ersten Analog-DigitalWandlers mit einem Ausgangsende des ersten Differenzverstärkers verbunden ist und so ausgelegt ist, dass er eine Analog-Digital-Wandlung an dem verstärkten Differenzwert zwischen den Spannungen durchführt und das EKG-Signal des ersten Kanals des Messobjekts erfasst.

13. Verfahren zur Überwachung des Elektrokardiogramms mit drei Kanälennach Anspruch 5, wobei die zweite Kanalschaltung Folgendes umfasst:

   einen zweiten Differenzverstärker, wobei zwei Eingangsenden des zweiten Differenzverstärkers jeweils mit der Antriebsleitungselektrode und einer der beiden Messleitungselektroden verbunden sind und so ausgelegt sind, dass sie einen Differenzwert zwischen den Spannungen der Messleitungselektrode und der Antriebsleitungselektrode verstärken, die mit dem zweiten Differenzverstärker verbunden sind und den verstärkten Differenzwert ausgeben; und
   einen zweiten Analog-Digital-Wandler, wobei ein Eingangsende des zweiten Analog-Digital-Wandlers mit einem Ausgangsende des zweiten Differenzverstärkers verbunden ist und so ausgelegt ist, dass er eine Analog-Digital-Wandlung an dem verstärkten Differenzwert zwischen den Spannungen durchführt und das EKG-Signal des zweiten Kanals des Messobjekts erfasst.

## Revendications

1. Procédé de surveillance d'électrocardiographie à trois dérivations, les trois dérivations comprenant deux électrodes de dérivation de mesure et une électrode de dérivation d'attaque qui sont fixées sur un objet de mesure, le procédé comprenant les étapes consistant à :

   acquérir un signal d'électrocardiographie d'un premier canal de l'objet mesuré au moyen des deux électrodes de dérivation de mesure ; et acquérir un signal d'électrocardiographie d'un second canal de l'objet mesuré au moyen de l'électrode de dérivation d'attaque et de l'une des deux électrodes de dérivation de mesure ; et **caractérisé en ce que** le procédé comprend en outre :
   le fait d'acquérir une amplitude d'interférence en mode commun dans un signal d'électrocardiographie de l'objet mesuré au moyen de l'un ou des deux des deux canaux ; et choisir, selon l'amplitude d'interférence en mode commun, l'un ou les deux du signal d'électrocardiographie acquis du premier canal et du signal d'électrocardiographie du second canal pour un traitement, afin d'obtenir un paramètre d'électrocardiographie de l'objet mesuré.

2. Procédé de surveillance d'électrocardiographie à trois dérivations selon la revendication 1, dans lequel lorsque l'amplitude d'interférence en mode commun est inférieure à un seuil, un choix est fait pour acquérir le signal d'électrocardiographie du premier canal et le signal d'électrocardiographie du second canal pour un traitement.

3. Procédé de surveillance d'électrocardiographie à trois dérivations selon la revendication 1, dans lequel lorsqu'il est déterminé que l'amplitude d'interférence en mode commun est supérieure ou égale à un seuil, un choix est fait pour acquérir uniquement le signal d'électrocardiographie du premier canal pour un traitement.

4. Procédé de surveillance d'électrocardiographie à trois dérivations selon la revendication 1, dans lequel des tensions des deux électrodes de dérivation de mesure sont moyennées, et une tension moyenne est fournie à l'électrode de dérivation d'attaque comme un premier signal d'attaque de jambe droite après avoir été inversée et amplifiée ; ou une tension de l'une des deux électrodes de dérivation de mesure est fournie à l'électrode de dérivation d'attaque comme le premier signal d'attaque de jambe droite après avoir été inversée et amplifiée.

5. Dispositif de surveillance d'électrocardiographie à trois dérivations, comprenant :

　　　deux électrodes de dérivation de mesure (01) et une électrode de dérivation d'attaque (02) qui sont fixées sur un objet mesuré ;
　　　un premier circuit de canal (10) relié aux deux électrodes de dérivation de mesure (01) et configuré pour acquérir un signal d'électrocardiographie d'un premier canal de l'objet mesuré au moyen des deux électrodes de dérivation de mesure (01) ;
　　　un second circuit de canal (20) relié à l'une des électrodes de dérivation de mesure (01) et à l'électrode de dérivation d'attaque (02), et configuré pour acquérir un signal d'électrocardiographie d'un second canal de l'objet mesuré au moyen de l'électrode de dérivation de mesure (01) et de l'électrode de dérivation d'attaque (02) qui sont reliées au second circuit de canal (20) ; et
　　　un processeur (30) ; **caractérisé en ce que** le processeur (30) est configuré pour acquérir une amplitude d'interférence en mode commun dans un signal d'électrocardiographie de l'objet mesuré au moyen de l'un ou des deux des deux canaux, et choisir, selon l'amplitude d'interférence en mode commun, l'un ou les deux du signal d'électrocardiographie acquis du premier canal et du signal d'électrocardiographie du second canal de l'objet mesuré pour un traitement, afin d'obtenir un paramètre d'électrocardiographie de l'objet mesuré.

6. Dispositif de surveillance d'électrocardiographie à trois dérivations selon la revendication 5, dans lequel lorsqu'il est déterminé que l'amplitude d'interférence en mode commun est inférieure à un seuil, le processeur (30) choisit d'acquérir le signal d'électrocardiographie du premier canal et le signal d'électrocardiographie du second canal pour un traitement.

7. Dispositif de surveillance d'électrocardiographie à trois dérivations selon la revendication 6, dans lequel le fait que le processeur (30) choisit d'acquérir le signal d'électrocardiographie du premier canal et le signal d'électrocardiographie du second canal pour un traitement comprend :
le processeur (30) est en outre configuré pour :

　　　générer, selon le signal d'électrocardiographie du premier canal de l'objet mesuré, une forme d'onde d'électrocardiogramme pour un affichage ; et/ou
　　　générer, selon le signal d'électrocardiographie du second canal de l'objet mesuré, une forme d'onde d'électrocardiogramme pour un affichage ; et/ou
　　　corriger le signal d'électrocardiographie de l'un des canaux de l'objet mesuré sur la base du signal d'électrocardiographie de l'autre canal ; et/ou
　　　déterminer, selon le signal d'électrocardiographie du premier canal et le signal d'électrocardiographie du second canal par analyse, si un électrocardiogramme de l'objet mesuré est normal.

8. Dispositif de surveillance d'électrocardiographie à trois dérivations selon la revendication 5, dans lequel lorsqu'il est déterminé que l'amplitude d'interférence en mode commun est supérieure ou égale à un seuil, le processeur (30) choisit d'acquérir uniquement le signal d'électrocardiographie du premier canal pour un traitement.

9. Dispositif de surveillance d'électrocardiographie à trois dérivations selon la revendication 8, dans lequel le fait que le processeur (30) choisit d'acquérir uniquement le signal d'électrocardiographie du premier canal pour un traitement comprend :
le processeur (30) est en outre configuré pour :

　　　générer, selon le signal d'électrocardiographie du premier canal de l'objet mesuré, une forme d'onde d'électrocardiogramme pour un affichage ; et/ou
　　　déterminer, selon le signal d'électrocardiographie du premier canal par analyse, si un électrocardiogramme de l'objet mesuré est normal.

10. Dispositif de surveillance d'électrocardiographie à trois dérivations selon la revendication 5, comprenant en outre :

une premier amplificateur-inverseur (41) relié à l'une des électrodes de dérivation de mesure (01) et configuré pour fournir une tension de l'électrode de dérivation de mesure reliée (01) qui est obtenue après inversion et amplification à l'électrode de dérivation d'attaque (02) comme un premier signal d'attaque de jambe droite ; ou un circuit de moyennage (42) et un second amplificateur-inverseur (43), où deux extrémités d'entrée du circuit de moyennage (42) sont respectivement reliées aux deux électrodes de dérivation de mesure (01), et sont configurées pour fournir en sortie une tension moyenne des deux électrodes de dérivation de mesure (01) au second amplificateur-inverseur (43) ; et le second amplificateur-inverseur (43) est configuré pour fournir la tension moyenne obtenue après inversion et amplification à l'électrode de dérivation d'attaque (02) comme un premier signal d'attaque de jambe droite.

11. Dispositif de surveillance d'électrocardiographie à trois dérivations selon la revendication 10, comprenant en outre :

une source de tension constante (44) reliée à l'électrode de dérivation d'attaque (02), et configurée pour fournir un signal de tension constante à l'électrode de dérivation d'attaque comme un second signal d'attaque de jambe droite ; et un commutateur, où lorsque le processeur choisit d'acquérir le signal d'électrocardiographie du premier canal et le signal d'électrocardiographie du second canal de l'objet mesuré pour un traitement, le processeur fournit le second signal d'attaque de jambe droite à l'électrode de dérivation d'attaque après une commutation effectuée au moyen du commutateur, ou fournit encore le premier signal d'attaque de jambe droite à l'électrode de dérivation d'attaque.

12. Dispositif de surveillance d'électrocardiographie à trois dérivations selon la revendication 5, dans lequel le premier circuit de canal comprend :

un premier amplificateur différentiel, deux extrémités d'entrée du premier amplificateur différentiel étant respectivement reliées aux deux électrodes de dérivation de mesure, et configuré pour amplifier une valeur de différence entre des tensions des deux électrodes de dérivation de mesure et fournir en sortie la valeur de différence amplifiée ; et un premier convertisseur analogique-numérique, une extrémité d'entrée du premier convertisseur analogique-numérique étant reliée à une extrémité de sortie du premier amplificateur différentiel, et configuré pour effectuer une conversion analogique-numérique sur la valeur de différence entre les tensions amplifiée, et acquérir le signal d'électrocardiographie du premier canal de l'objet mesuré.

13. Dispositif de surveillance d'électrocardiographie à trois dérivations selon la revendication 5, dans lequel le second circuit de canal comprend :

un second amplificateur différentiel, deux extrémités d'entrée du second amplificateur différentiel étant respectivement reliées à l'électrode de dérivation d'attaque et à l'une des deux électrodes de dérivation de mesure, et configuré pour amplifier une valeur de différence entre des tensions de l'électrode de dérivation de mesure et de l'électrode de dérivation d'attaque qui sont reliées au second amplificateur différentiel, et fournir en sortie la valeur de différence amplifiée ; et un second convertisseur analogique-numérique, une extrémité d'entrée du second convertisseur analogique-numérique étant reliée à une extrémité de sortie du second amplificateur différentiel, et configuré pour effectuer une conversion analogique-numérique sur la valeur de différence entre les tensions amplifiée, et acquérir le signal d'électrocardiographie du second canal de l'objet mesuré.

**FIG. 1**

**FIG. 2**

**FIG. 3**

**FIG. 4**

Measured object

First analog-to-digital converter 12

First channel

First analog-to-digital converter 22

Second channel

Processor

*FIG. 5*

Measured object

R1

First channel circuit 10

First channel

Second channel circuit 20

Second channel

Processor

R 41

*FIG. 6 (a)*

Measured object

R1

R2

Averaging circuit 42

First channel circuit 10

First channel

Second channel circuit 20

Second channel

Processor

R 43

*FIG. 6 (b)*

FIG. 6 (c)

FIG. 7

**EP 3 821 799 B1**

```
┌─────────────────────────────────────┐
│  Constructing an electrocardiography │  ⌇310
│      signal of a first channel       │
└─────────────────────────────────────┘
                  │
                  ▼
┌─────────────────────────────────────┐
│  Constructing an electrocardiography │  ⌇320
│     signal of a second channel       │
└─────────────────────────────────────┘
                  │
                  ▼
┌──────────────────────────────────────────────────┐
│ Choosing to acquire an electrocardiography signal │  ⌇330
│    of one or two channels for processing          │
└──────────────────────────────────────────────────┘
```

*FIG. 8*

```
                  ╱◇╲
            ╱  Is a common-  ╲           No
      ╱ mode interference amplitude greater than or ╲ ────────┐
      ╲         equal to a threshold?          ╱             │
            ╲                             ╱                   │
                  ╲◇╱                                         │
                  │ Yes                                       │
                  ▼                                           │
┌──────────────────────────────────────────────────┐         │
│ Choosing to acquire only an electrocardiography    │         │
│    signal of a first channel for processing        │         │
└──────────────────────────────────────────────────┘         │
                                                              │
                                                              │
┌──────────────────────────────────────────────────┐         │
│ Choosing to acquire electrocardiography signals of │◄────────┘
│  the first channel and a second channel for        │
│                processing                          │
└──────────────────────────────────────────────────┘
```

*FIG. 9*

22

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0835635 A
- US 20100152596 A
- US 20070038257 A